# EUROPÄISCHE PATENTANMELDUNG

(11) **EP 1 923 041 A1**
(43) Veröffentlichungstag der Anmeldung: **21.05.2008**
(21) Anmeldenummer: 06122667.6
(22) Anmeldetag: 20.10.2006
(51) Int. Cl.: A61K 8/23, A61K 8/26, A61K 8/31, A61K 8/34, A61K 8/35, A61K 8/42, A61K 8/49, A61K 8/96, A61Q 5/00

(54) **Verwendung von C10-C14-Alkandiolen zur Herstellung eines Mittels zur Prophylaxe und/oder Behandlung von Malassezia-induzierter Schuppenbildung, sowie Zubereitungen enthaltend C10-C14-Alkandiole**

(71) Anmelder: Symrise GmbH & Co. KG, 37603 Holzminden (DE)
(72) Erfinder: Schmaus, Gerhard, 37671, Höxter (DE); Lange, Sabine, 37603 Holzminden (DE)
(74) Vertreter: Eisenführ, Speiser & Partner

(57) **Zusammenfassung**

Die Erfindung betrifft die Verwendung von C10-C14-Alkan-1,2-diolen, insbesondere von 1,2-Decandiol, 1,2-Dodecandiol und/oder 1,2-Tetradecandiol zur Herstellung eines Mittels zur Prophylaxe und/oder Behandlung von *malassezia*-induzierter Schuppenbildung sowie kosmetische und/oder dermatologische Zubereitungen umfassend C10-C14-Alkan-1,2-diole.

## Beschreibung

Die Erfindung betrifft die Verwendung von ein, zwei, drei oder mehreren C10-C14-Alkan-1,2-diolen, insbesondere von 1,2-Decandiol, 1,2-Dodecandiol und/oder 1,2-Tetradecandiol zur Herstellung eines Mittels zur Prophylaxe und/oder Behandlung von Malassezia-induzierter Schuppenbildung sowie kosmetische und/oder dermatologische Zubereitungen umfassend ein, zwei, drei oder mehrere C10-C14-Alkan-1,2-diole.

Kopfhautschuppen sind eine der häufigsten Störungen der Haut von der weltweit mehr als 50% der Männer und Frauen betroffen sind. Die Bildung von Kopfhautschuppen kann unter anderem durch folgende Faktoren ausgelöst werden:
- durch Erbanlagen verursachte Funktionsstörungen der Talgdrüsen,
- durch die Erkrankung innerer Organe oder die Störung ihrer Funktion,
- durch Nebenwirkung von Medikamenten,
- durch Stress, Überbelastung der Nerven und psychische Probleme,
- durch Auslaugen und Austrocknen der Kopfhaut durch ungeeignete Pflegemittel, Bakterien und Pilze sowie
- durch erhöhte Sebumproduktion der Kopfhaut.

Kopfschuppen entstehen dabei zumeist als Folge einer Überproduktion von Hornzellen. Diese Überproduktion wird ausgelöst durch winzige, nur mit dem Mikroskop sichtbare Entzündungsherde der Kopfhaut. Durch die Entzündung reifen die Hornzellen der Kopfhaut nur unvollständig aus, so dass sie verfrüht in großen Zellverbänden - als Schuppen - abschilfern.

Die Ursache für die Schuppenbildung ist häufig eine vermehrte Keimbesiedelung mit Bakterien oder Pilzen. Malassezia-Arten, wie z. B. *Malassezia furfur* oder *Malassezia globosa*, sind hierbei von besonderer Relevanz. Malassezia-Arten ernähren sich von gesättigten Fettsäuren, die sie mittels eigens freigesetzter Lipasen durch Abbau der im menschlichen Sebum der Haut vorkommenden Triglyceride gewinnen. Beim Triglycerid-Abbau werden jedoch auch große Mengen ungesättigter Fettsäuren freigesetzt, die vom Pilz nicht metabolisiert werden, sich dadurch im Sebum stark anreichern und infolge dessen zum Teil starke Kopfhaut-Irrtiationen verursachen. Das vermehrte Wachstum von Malassezia Arten, z. B. wie M. globosa und *M. furfur* kann somit als eine der Hauptursachen für Schuppenbildung einhergehend mit starker Rötung sowie mit starkem Juckreiz der Kopfhaut betrachtet werden. Kopfhautrötung und Juckreiz können durch vermehrte Sebumproduktion (Seborrhoe) noch weiter gefördert werden.

Bei der Behandlung einer erhöhten Schuppenbildung bemüht man sich darum beispielsweise die Schuppen abzulösen, die Überproduktion von Hornzellen zu vermindern und/oder das Wachstum von Pilzen zu inhibieren. Zur Anwendung kommen dabei u.a. salicylsäurehaltige Therapeutika, Antimykotika mit guter Wirksamkeit gegen Malassezia-Arten, Teerhaltige Externa, Schwefel und/oder Selendisulfid.

Die Inhibition des Wachstums von Pilzen der Gattung *Malassezia* stellt dabei eines der Hauptverfahren zur Bekämpfung von Schuppenbildung dar. Eine wichtige Gruppe von Wirkstoffen, die aktuell zur Behandlung von Schuppen eingesetzt werden, sind topisch sowie systemisch anzuwendende Antimykotika aus der Gruppe der Azole. Bevorzugte Antischuppen-Mittel hiervon sind Climbazol sowie weitere Azole wie z.B. Benzimidazol, Benzothiazol, Bifonazol, Butaconazolnitrat, Clotrimazol, Croconazol, Eberconazol, Econazol, Elubiol, Fenticonazol, Fluconazol, Flutimazol, Isoconazol, Ketoconazol, Lanoconazol, Metronidazol, Miconazol, Neticonazol, Omoconazol, Oxiconazolnitrat, Sertaconazol, Sulconazolnitrat, Thioconazol sowie Diazole und Triazole wie z.B. Terconazol und Itraconazol sowie beliebige Kombinationen der genannten Azole. Insbesondere aufgrund des in der klinischen Praxis häufiger zu beobachtenden Auftretens von Sensiblisierungs-Reaktionen aber auch der Bildung potentiell resistenter Stämme geraten die in der Schuppenbehandlung geläufigen Antimykotika jedoch zunehmend ins Licht der Diskussion.

In Antischuppen-Mitteln werden im Weiteren spezielle organische Carbonsäuren wie z. B. Salicylsäure eingesetzt. Bedingt durch die zum Teil sehr hohe Dosierung wird jedoch die Haut sehr stark strapaziert, was sich insbesondere in starker Austrocknung der Haut äußert, die zum Teil aufgrund der starken Reduktion des pH-Wertes der Haut ebenfalls mit starken Hautirritationen einhergehen kann.

Als weiteres Antischuppen-Mittel wird Steinkohleteer eingesetzt, der jedoch aufgrund kanzerogener Nebenwirkungen ebenfalls in die Kritik geraten ist.

Es war daher die Aufgabe der vorliegenden Erfindung eine Verbindung bereit zu stellen, die eine Wirksamkeit gegenüber Malassezia-Arten aufweist und zugleich mild und hautverträglich ist, insbesondere nicht hautreizend, nicht ätzend, nicht sensibilisierend und/oder nicht kanzerogen ist sowie bei einem hautfreundlichen pH-Wert wirkt.

Die Aufgabe wird gelöst zumindest teilweise durch die Gegenstände der vorliegenden Erfindung, insbesondere wie sie in den vorliegenden Ansprüchen beschrieben werden.

Eine Ausgestaltung der vorliegenden Erfindung betrifft die Verwendung von ein, zwei, drei oder mehreren C-10-C14-Alkan-1,2-diolen zur Herstellung eines Mittels zur Prophylaxe und/oder Behandlung von malassezia-induzierter Schuppenbildung.

Eine weitere Ausgestaltung der vorliegenden Erfindung betrifft eine topisch zu applizierende, kosmetische und/oder dermatologische Zubereitung umfassend oder bestehend aus:
a) einer Schuppen-reduzierenden Menge an ein, zwei, drei, vier oder mehreren C10-C14-Alkan-1,2-Diolen,
b) einer Schuppen-reduzierenden Menge an ein, zwei, drei, vier oder mehreren Antischuppen-Mitteln ausgewählt aus der Gruppe bestehend aus Climbazol, Benzimidazol, Benzothiazol, Bifonazol, Butaconazolnitrat, Clotrimazol, Croconazol, Eberconazol, Econazol, Elubiol, Fenticonazol, Fluconazol, Flutimazol, Isoconazol, Ketoconazol, Lanoconazol, Metronid azol, Miconazol, Neticonazol, Omoconazol, Oxiconazolnitrat, Sertaconazol, Sulconazolnitrat, Thioconazol, Itraconazol, Zinkpyrithion, Steinkohleteer, Schwefel, Selendisulfid, Aluminumchlorid, Octopirox, Cyclopiroxolamine, Undecylensäure und deren Metallsalze, Harnstoffderivate, Griseofulvin, 8-Hydroxyquinolin, Ciloquinol, Thiobendazol, Thiocarbamate, Triclosan, Haloprogin, Polyene, Hydroxypyridon, Morpholin, Benzylamin, Allylamine, Teebaumöl, Nelkenblätteröl, Korianderöl, Palmarosaöl, Thymianöl, Zimtöl sowie etherisches Öl der Bitterorange, Zimtaldehyd, Citronellsäure, Farnesol, Berberin, Hinokitiol, Tropolon, Birkenteeröle, Ichthyol, Sensiva SC-50, Polyglycerolester, Arylalkylalkohole, 3-Phenyl-1-propanol, Vetikol, Muguetalkohol, Elestab HP-100, Azelainsäure, Lyticas, Isothiazolinone und lodopropinylbutyl-carbamat und
c) gegebenenfalls ein, zwei oder mehreren Hilfs- und/oder Zusatzstoffen.

Eine weitere Ausgestaltung der vorliegenden Erfindung betrifft eine Duftstoffkomposition umfassend oder bestehend aus:
a) eine sensorisch wirksame Menge eines Riechstoffes oder der Summe aus zwei, drei, vier oder mehreren Riechstoffen,
b) eine Schuppen-reduzierenden Menge eines, zweier, dreier oder mehrerer, bevorzugt eines, zweier oder dreier erfindungsgemäßer C10-C14-Alkan-1,2-diole, weiter bevorzugt einer Verbindung oder einer Mischung aus zwei oder drei Verbindungen ausgewählt aus der Gruppe bestehend aus 1,2-Decandiol, 1,2-Dodecandiol und 1,2-Tetradecandiol und
c) gegebenenfalls ein oder mehreren Träger- und/oder Zusatzstoffen.

Im Sinne der vorliegenden Erfindung weisen die C10-C14-Alkan-1,2-Diole eine unverzweigte Alkankette mit 10 bis 14 Kohlenstoffatomen auf, wobei die OH-Substituten in Position1 und 2 vorhanden sind, und es sind sowohl (a) 2S-konfigurierte Enantiomere als auch (b) 2R-konfigurierte Enantiomere sowie (c) beliebige Mischungen aus 2S- und 2R-konfigurierten C10-C14-Alkan-1,2-diolen umfasst. Aus kommerziellen Gründen ist es zwar besonders vorteilhaft, Razemate von C10-C14-Alkan-1,2-diolen zur Prophylaxe und/oder Behandlung von malassezia-induzierter Schuppenbildung einzusetzen, da diese synthetisch besonders leicht zugänglich sind, die reinen Enantiomere oder nicht-razemische Mischungen dieser Enantiomere sind aber ebenfalls für die erfindungsgemäßen Zwecke bevorzugt geeignet.

Die Verbesserung des Zustandes zu malassezia-induzierter Schuppenbildung neigender Haut beruht dabei, wie die Untersuchung der biologischen Eigenschaften von Alkan-1,2-diolen zeigten (vgl. Bsp. 1), im wesentlichen auf der Eigenschaft der erfindungsgemäßen C10-C14-Alkan-1,2-diole das Wachstum der an Schuppenbildung maßgeblich beteiligten Pilze der Gattung *Malassezia* (z. B. *Malassezia furfur;* Synonym: *Pityrosporum ovale)* zu inhibieren.

Bevorzugt ist eine erfindungsgemäße Verwendung von einem oder mehreren, bevorzugt einem, zwei, drei oder mehreren C10-C14-Alkan-1,2-diolen weiter bevorzugt eine Verbindung oder eine Mischung aus zwei oder drei Verbindungen ausgewählt aus der Gruppe bestehend aus 1,2-Decandiol, 1,2-Dodecandiol und 1,2-Tetradecandiol.

Der Stand der Technik bezüglich der antimikrobiellen Wirkung aliphatischer 1,2-Diole im allgemeinen und insbesondere deren Einsatzmöglichkeiten zur Inhibition des Wachstums von Vertretern der Gattung *Malassezia* wie z. B. *Malassezia furfur* (Synonym: *Pityrosporum ovale)* wird im folgenden näher beschrieben.

Zwar ist die antimikrobielle Wirkung aliphatischer Alkan-1,2-diole an sich bereits hinlänglich bekannt, allerdings wird keine Wirkung der Inhibition von Malassezia-Arten beschrieben.

So sind aus JP 51 91327 bestimmte Hinweise zur bakteriostatischen Wirkung von Alkan-1,2-diolen gegenüber bestimmten Keimen im Zusammenhang mit der Konservierung von Lebensmitteln und Kosmetika bekannt, wohingegen jedoch keine antimikrobielle Wirkung der Alkan-1,2-diole gegenüber Malassezia-Arten (z. B. *Malassezia furfur;* Synonym: *Pityrosporum ovale),* den bei Schuppenbildung eine Rolle spielenden Keimen offenbart wird.

Zudem beschreibt JP 2002/2003330 die antibakterielle Wirkung einer Kombination von 1,2-Alkandiolen und Ascorbinsäureestern, wohingegen jedoch auch in dieser Schrift keine antimikrobielle Wirkung der Alkan-1,2-Diole gegenüber Malassezia-Arten (z. B. *Malassezia furfur;* Synonym: *Pityrosporum ovale)* den bei Schuppenbildung eine Rolle spielenden Keimen offenbart wird.

Aus JP 11 322591 ist lediglich bekannt, dass bestimmte 1,2-Alkandiole mit einer Kettenlänge von 4 bis 10 C-Atomen eingesetzt werden können, um die Dosis konventioneller antiseptischer Mikrobizide in einer antimikrobiellen Formulierung zu reduzieren. Als bevorzugt werden 1,2-Pentandiol und 1,2-Hexandiol sowie 1,2-Octandiol genannt. Ein Hinweis auf die Wirkung von Alkan-1,2-Diolen gegenüber Malassezia-Arten (z. B. *Malassezia furfur;* Synonym: *Pityrosporum ovale)* die an Schuppenbildung maßgeblich beteiligt sind, ist in der japanischen Schrift hingegen nicht enthalten.

In FR 2,771,632 ist offenbart, dass bestimmte Alkan-1,2-diole mit einer Kettenlänge von 8 bis 18 C-Atomen im Gemisch mit einer N-Acylaminosäure als Mittel gegen Akne und Schuppenbildung sowie zur Desinfektion von Haut und Schleimhaut eingesetzt werden können. Als bevorzugt wird hier überraschenderweise 1,2-Octandiol genannt.

US 4,294,728 beschreibt 1,2-Diole als Mittel zur Verbesserung der Schaumcharakteristik, wodurch eine bessere Haarentwirrung erreicht wird. Ein Hinweis auf die Wirkung von Alkan-1,2-Diolen gegenüber Malassezia-Arten (z. B. *Malassezia furfur;* Synonym: *Pityrosporum ovale),* die an Schuppenbildung maßgeblich beteiligt sind, ist in dieser Schrift hingegen nicht enthalten.

EP 0 584 692 betrifft Mittel zur Reinigung und Konditionierung der Haare, der Haut usw., welche Alkan-1,2-Diole mit einer Kettenlänge von C10 bis C16 enthalten. Die dort beschriebenen Mittel zeigen verbesserte Schaumeigenschaften und ein besseres Hautgefühl und eignen sich ebenfalls hervorragend als Rückfettungsmittel (Konditioniermittel). Ein Hinweis auf die Wirkung von Alkan-1,2-Diolen gegenüber Malassezia-Arten (z. B. *Malassezia furfur;* Synonym: *Pityrosporum ovale*), die an Schuppenbildung maßgeblich beteiligt sind, ist in dieser Schrift hingegen nicht enthalten.

WO 00/00164 beschreibt lediglich Alkan-1,2-diole mit einer Kettenlänge von C5 bis C8 zur Verringerung von krausem Haar (Glättung der Haare). Ein Hinweis auf die Wirkung von Alkan-1,2-Diolen gegenüber Malassezia-Arten (z. B. *Malassezia furfur;* Synonym: *Pityrosporum ovale),* die an Schuppenbildung maßgeblich beteiligt sind, ist in dieser Schrift ebenfalls nicht enthalten.

Die WO 03/000220 (Dragoco Gerberding GmbH & Co. KG) beschreibt lediglich die Verwendung von 1,2-Decandiol gegen Körpergeruch verursachende Bakterien. Ein Hinweis auf die Wirkung von Alkan-1,2-Diolen gegenüber Malassezia Arten (z. B. *Malassezia furfur;* Synonym: *Pityrosporum ovale),* die an Schuppenbildung maßgeblich beteiligt sind, ist auch in dieser Schrift nicht enthalten.

Die EP 0 524 548 offenbart bestimmte antimikrobiell wirksame Gemische, die neben (A) einem antimikrobiell wirksamen aromatischen Alkohol der Formel 1, 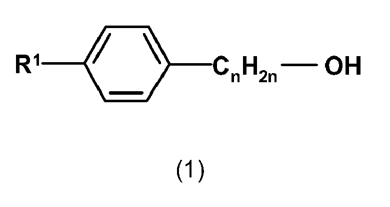 in der R¹ Wasserstoff oder eine Alkylgruppe mit 1 bis 4 C-Atomen und n eine ganze Zahl von 1 bis 6 ist, (B) ein antimikrobiell wirksames 1,2- oder 1,3-Diol der Formel R²-CHOH-(CHR³)ₓ-CH₂OH enthalten, wobei x = 0 oder 1 ist und wenn x = 0 ist, R² eine Alkylgruppe mit 6 bis 22 C-Atomen oder eine Alkoxymethyl- oder 2-Hydroxyalkoxymethylgruppe mit jeweils 6 bis 22 C-Atomen in der Alkoxygruppe ist, und wenn x = 1 ist, die Gruppe R² Wasserstoff ist und R³ eine der vorgenannten Bedeutungen von R² hat, wobei die Komponenten im Gewichtsverhältnis 9:1 bis 1:9 vorliegen. Die offenbarten antimikrobiell wirksamen Gemische werden als geeignet zur Herstellung antiseptisch wirksamer Hautreinigungsmittel und zur Konservierung wässriger Zubereitungen mikrobiell abbaubarer oder verderblicher Stoffe bezeichnet. Ein Hinweis auf eine Wirkung von Alkan-1,2-Diolen gegenüber speziellen an der Schuppenbildung beteiligten Pilzen wie insbesondere Malassezia Arten (z. B. *Malassezia furfur,* Synonym: *Pityrosporum ovale)* ist auch in EP 0 524 548 nicht enthalten.

In der US 6,123,953 wird ausgeführt, dass unverzweigte 1,2-Alkandiole mit einer Kettenlänge von insgesamt 5 bis 14 C-Atomen und vorzugsweise 6 bis 8 C-Atomen zur topischen Applikation auf die Haut und hierbei insbesondere zur Behandlung von Akne, zur Behandlung von Kapillarschichten, zur Desinfektion kleiner Wunden (Kratzer), zur Desinfektion der Hände von Chirurgen und Kranken sowie zur Desinfektion der Euter von milchgebenden Tieren geeignet sind. US 6,123,953 offenbart ferner, dass Formulierungen, die neben einem Alkandiol ein Gel des Glyceryl-Polymethacrylat-Typs enthalten, in niedrigen Konzentration zur Behandlung von Akne, Hautschwierigkeiten, Impetigo, Mikroorganismen basierten Körpergerüchen, "athlete's food" und dergleichen eingesetzt werden können. Diese Einsetzbarkeit wird dabei auf eine synergistische Wechselwirkung zwischen dem Gel und dem Alkandiol zurückgeführt. Als bevorzugtes Alkandiol zur Behandlung von Akne wird in dieser Schrift jedoch 1,2-Octandiol angegeben. Ein Hinweis auf eine Wirkung von Alkan-1,2-Diolen, insbesondere von Alkan-1,2-diolen mit einer Kettenlänge von C10 bis C14 gegenüber speziellen an der Schuppenbildung beteiligten Pilze wie insbesondere *Malassezia furfur* (Synonym: *Pityrosporum ovale)* ist auch in US 6,123,953 nicht enthalten.

EP 1 598 064 offenbart den Einsatz von 1,2-Decandiol in Kombination mit alpha-Hydroxysäuren in Anti-Akne Produkten. Ein Hinweis auf eine Wirkung von Alkan-1,2-Diolen gegenüber speziellen an der Schuppenbildung beteiligten Pilze wie insbesondere *Malassezia furfur* (Synonym: *Pityrosporum ovale)* ist auch in EP 1 598 064 nicht enthalten.

Gemäß der vorliegenden Erfindung können ein, zwei, drei oder mehrere, bevorzugt ein, zwei oder drei C10-C14-Alkan-1,2-diole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol ausgewählt werden. Die bevorzugten Alternativen der C10-C14-Alkan-1,2-diole sind auf alle Gegenstände der vorliegenden Erfindung anzuwenden.

Bevorzugt ist ebenfalls eine erfindungsgemäße Zubereitung, dadurch gekennzeichnet, dass ein, zwei oder mehrere Hilfs- und/oder Zusatzstoffe aus der Gruppe der Riechstoffe ausgewählt werden. Es hat sich als besonders vorteilhaft erwiesen, dass die erfindungsgemäßen C10-C14-Alkan-1,2-diole, insbesondere 1,2-Decandiol, 1,2-1,2-Dodecandiol und/oder 1,2-Tetradecandiol jeweils nur einen schwachen Eigengeruch besitzen oder gar geruchlos sind und dadurch prädestiniert dafür sind, den Einsatz der erfindungsgemäßen Duftstoffkomposition nicht zu stören.

Die erfindungsgemäßen C10-C14-Alkan-1,2-diole werden insbesondere vorteilhaft in kosmetischen und/oder dermatologischen Zubereitungen verwendet. Es ist dabei von Vorteil, wenn in den kosmetischen und/oder dermatologischen Zubereitungen der Gehalt an einem, zwei, drei oder mehreren, bevorzugt ein, zwei oder drei erfindungsgemäßen C10-C14-Alkan-1,2-diolen in einer Gesamtkonzentration von 0,1 bis 10 Gew.-%, bevorzugt in einer Konzentration von 0,2 bis 5 Gew.-% und ganz besonders bevorzugt in einer Konzentration von 0,5 bis 4 Gew.-%, jeweils bezogen auf das Gesamtgewicht der Zubereitung eingesetztwerden.

Bevorzugt werden ein, zwei oder mehrere Antischuppen-Mittel zusätzlich zu einem oder mehreren, bevorzugt zu einem, zwei oder drei C10-C14-Alkan-1,2-diolen, weiter bevorzugt zu einer Verbindung oder einer Mischung aus zwei oder drei Verbindungen aus der Gruppe bestehend aus 1,2-Decandiol, 1,2-Dodecandiol und 1,2-Tetradecandiol in einer erfindungsgemäßen Zubereitung verwendet.

Ein, zwei oder mehrere weitere Antischuppen-Mittel werden bevorzugt ausgewählt aus der Gruppe der Azole bestehend aus Climbazol, Benzimidazol, Benzothiazol, Bifonazol, Butaconazolnitrat, Clotrimazol, Croconazol, Eberconazol, Econazol, Elubiol, Fenticonazol, Fluconazol, Flutimazol, Isoconazol, Ketoconazol, Lanoconazol, Metronidazol, Miconazol, Neticonazol, Omoconazol, Oxiconazolnitrat, Sertaconazol, Sulconazolnitrat, Thioconazol sowie Diazole und Triazole, bevorzugt Terconazol und Itraconazol sowie beliebige Kombinationen der genannten Azole.

Alternativ oder kumulativ zu den Azolen können bevorzugt auch Pyrithionsalze, bevorzugt1-Hydroxy-2-pyrithionsalze, als Antischuppen-Mittel in Kombination mit ein oder mehreren erfindungsgemäßen C10-C14-Alkan-1,2-diolen zur Behandlung von Schuppenbildung eingesetzt werden. Bevorzugt sind hierbei Pyrithionsalze der Metallkationen von Natrium, Zink, Zinn, Cadmium, Magnesium, Aluminium und Zirkonium. Besonders bevorzugt ist das Zinksalz von 1-Hydroxy-2-pyrithion (bekannt als "Zinkpyrithion" oder "ZPT").

Alternativ oder kumulativ zu den Azolen und/oder Pyrithionsalze können ein oder mehrere Antischuppen-Mittel bevorzugt aus der Gruppe ausgewählt werden bestehend aus Steinkohleteer; Schwefel; Selendisulfid; Aluminumchlorid; Octopirox (INCI: Piroctone Olamine); Cyclopiroxolamine; Undecylensäure und deren Metallsalze; Kaliumpermanganat; Natriumthiosulfat, Harnstoffzubereitungen, Griseofulvin, 8-Hydroxyquinolin, Ciloquinol, Thiobendazol; Thiocarbamate; Triclosan; Haloprogin; Polyene; Hydroxypyridon; Morpholin; Benzylamin; Allylamine, bevorzugt Terbinafin;Teebaumöl; Nelkenblätteröl; Korianderöl; Palmarosaöl; Thymianöl; Zimtöl sowie etherisches Öl der Bitterorange; Zimtaldehyd; Citronellsäure; Farnesol; Berberin; Hinokitiol; Tropolon; Birkenteeröle; Ichthyol(sulphoniertes Schieferöl) Sensiva SC-50 (Etyhlexylglycerin); Polyglycerolester, bevorzugt Polyglycerol-3-caprylat; Arylalkylalkohole, bevorzugt Phenylethylalkohol; 3-Phenyl-1-propanol; Vetikol,(4-Methyl-4-phenyl-2-pentanol); Muguetalkohol 2,2 -Dimethyl-3-phenylpropanol; Elestab HP-100; Azelainsäure; Lyticas; Isothiazolinone, bevorzugt Pctylisothiazolinon und lodopropinylbutyl-carbamat.

Der Fachmann kann die vorstehende Liste um eine Vielzahl weiterer Antischuppen-Mittel ergänzen; die aufgelisteten Antischuppen-Mittel können auch in Kombination miteinander eingesetzt werden. Bevorzugt werden ein, zwei, drei oder mehrere zusätzliche Antischuppen-Mittel in Kombination mit ein oder mehreren, bevorzugt ein, zwei oder drei erfindungsgemäßen C10-C14-Alkandiolen, weiter bevorzugt mit einer Verbindung oder einer Mischung aus zwei oder drei Verbindungen aus der Gruppe bestehend aus 1,2-Decandiol, 1,2-Dodecandiol und 1,2-Tetradecandiol in Zubereitungen zur Behandlung von Schuppenbildung eingesetzt, wobei das oder die zusätzlichen Antischuppen-Mittel ausgewählt werden aus der Gruppe bestehend aus: Climbazol, Ketoconazol, Zinkpyrithion, Schwefel, Selendisulfide, Octopirox (INCI: Piroctone Olamine), Cyclopiroxolamin, Undecylensäure und deren Metallsalze, Ichthyol (sulphoniertes Schieferöl), Sensiva SC-50 (Etyhlhexylglycerin) und/oder Vetikol (4-Methyl-4-phenyl-2-pentanol).

Da Schuppenbildung zumeist mit entzündlichen Prozessen einhergeht, was sich insbesondere in starker Rötung und Jucken der Kopfhaut äußert, können kosmetische Zubereitungen, die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol enthalten, besonders vorteilhaft auch ein, zwei oder mehrere entzündungshemmende und/oder rötungs-und/oder juckreizlindernde Wirkstoffe (Entzündungshemmer) enthalten. Es können hierbei alle für kosmetische und/oder dermatologische Anwendungen geeigneten oder gebräuchlichen entzündungshemmenden bzw. rötungs- und/oder juckreizlindernden Wirkstoffe verwendet werden.

Vorteilhaft werden als entzündungshemmende bzw. rötungs- und/oder juckreizlindernde Wirkstoffe ein, zwei, drei oder mehrere steroidale entzündungshemmende Wirkstoffe vom Kortikosteroiden-Typ eingesetzt, bevorzugt Hydrocortison, Hydrocortison-Derivate, wie Hydrocortison-17-butyrat, Dexamethason, Dexamethasonphosphat, Methylprednisolon oder Cortison, wobei die Auflistung durch Zusatz weiterer steroidaler Entzündungshemmer erweiterbar ist.

Alternativ oder kumulativ können ein, zwei, drei oder mehrere nichtsteroidale Entzündungshemmer eingesetzt werden, ausgewählt aus der Gruppe bestehend aus Oxicame, bevorzugt Piroxicam oder Tenoxicam; Salicylate, bevorzugt Aspirin, Disalcid, Solprin oder Fendosal; Essigsäre-Derivate, bevorzugt Diclofenac, Fenclofenac, Indomethacin, Sulindac, Tolmetin, oder Clindanac; Fenamate, bevorzugt Mefenamic, Meclofenamic, Flufenamic oder Niflumic; Propionsäure-Derivate, bevorzugt Ibuprofen, Naproxen, Benoxaprofen oder Pyrazole wie Phenylbutazon, Oxyphenylbutazon, Febrazon oder Azapropazon.

Alternativ oder kumulativ können natürlich vorkommende entzündungshermmende bzw. rötungs- und/oder juckreizlindernde Wirkstoffe eingesetzt werden. Einsetzbar sind Pflanzenextrakte, spezielle hochwirksame Pflanzenextrakt-Fraktionen sowie aus Pflanzenextrakten isolierte hochreine Wirksubstanzen. Besonders bevorzugt sind Extrakte, Fraktionen und Wirksubstanzen aus Kamille, Aloe vera, Commiphora-Arten, Rubia-Arten, Weiden, Weidenröschen, Hafer, Calendula, Arnika, Johanniskraut, Geißblatt, Rosmarin, Melisse, Ingwer, Passiflora incarnata, Hamamelis, Pueraria, Dianthus oder Echinacea sowie Reinsubstanzen wie u.a. Bisabolol, Apigenin, Apigenin-7-glucosid, Rosmarinsäure, Boswelliasäure, Phytosterole, Glycyrrhizinsäure, Glabridin, Licochalkon A, Gingerole und Anthranilsäureamide, bevorzugt insbesondere Avenanthramid oder Dianthramid.

Die Menge der entzündungshemmenden bzw. rötungs- und/oder juckreizlindernden Wirkstoffe (eine oder mehrere Verbindungen) in den Zubereitungen beträgt vorzugsweise 0,0001 bis 20 Gew.-%, besonders bevorzugt 0,0001 - 10 Gew.-%, insbesondere 0,001 - 5 Gew.-%, jeweils auf das Gesamtgewicht der Zubereitung.

Ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol können dabei in kosmetischen und/oder dermatologischen Formulierungen unterschiedlichster Formen zum Einsatz gelangen. So können sie z.B. eine Lösung (z. B. wässrige, wässrig-alkoholische oder alkoholische Lösung), eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O) (jeweils auch in Form von Silikonöl-Emulsionen), eine Hydrodispersion oder Lipodispersion, eine Pickering-Emulsion, einen festen Stift oder auch ein Aerosol darstellen. Auch die Tränkung eines wasserunlöslichen Substrats mit erfindungsgemäßen C10-C14-Alkan-1,2-diolen (z.B. ein Tuch, ein Vlies, ein Pad) kann vorteilhaft sein, wobei es sich bei dieser Applikationsform sowohl um ein trocken anmutendes als auch um ein feucht anmutendes Substrat handeln kann. Weitere vorteilhafte Darreichungsformen der erfindungsgemäßen C10-C14-Alkan-1,2-diole sind Cremes, Salben, Hydrodispersionen, Lotionen, Tinkturen, Pumpsprays, Aerosolsprays, wässrige Lösungen, Reinigungssubstrate und dergleichen. Die wässrige Phase kann dabei neben Wasser auch andere Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, - monoethyl- oder -monobutylether, Diethylenglykol- monomethyl oder monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die erfindungsgemäßen C10-C14-Alkan-1,2-diole lassen sich mit einer Vielzahl weiterer Hilfs- und/oder Zusatzstoffe kombinieren, wodurch sich bevorzugte kosmetische und/oder pharmazeutische Mischungen bzw. Produkte ergeben.

### Kombination mit Anti-Akne Produkten:

Kosmetische und/oder dermatologische Zubereitungen die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, enthalten, können zusätzlich ein, zwei oder mehrere gängige Anti-Akne Wirkstoffe enthalten, bevorzugt ausgewählt aus der Gruppe bestehend aus Benzoylperoxid, Azelainsäure, Salicylsäure, Retinoide wie all-trans-Retinsäure (Tretinoin), all-trans-Retinal oder cis-13-Retinsäure (Isotretinoin) sowie Antibiotika, die speziell zur Behandlung von Akne zum Einsatz gelangen wie z.B. Clindamycin, Erythromycin, Tetracyclin, Doxycycline und/oder Schwefel. Auch Chlorhexidin, Triclosan, Bisabolol, Farnesol und Phenoxyethanol sowie Isoflavonoide (z.B. Genistein, Daidzein, Genistin und Glycitein) können ebenfalls vorteilhaft gemeinsam mit ein oder mehrere, bevorzugt ein, zwei oder drei erfindungsgemäßen C10-C14-Alkan-1,2-diolen zur Prophylaxe und/oder Behandlung von malassezia-induzierter Schuppenbildung eingesetzt werden.

### Kombination mit Hautfeuchte regulierenden Produkten:

Ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, lassen sich besonders vorteilhaft zusätzlich mit ein, zwei, drei oder mehreren Hautfeuchte regulierenden Verbindungen kombinieren. Kosmetische Zubereitungen, die erfindungsgemäße C10-C14-Alkan-1,2-diole enthalten, können daher vorteilhafterweise zusätzlich ein, zwei, drei oder mehrere Feuchthalteregulatoren enthalten, ausgewählt aus der Gruppe bestehend aus: Natriumlactat; Harnstoff; Harnstoffderivate; Alkohole, bevorzugt Glycerin, weitere Diole wie Propylenglykol, Hexylenglycol, 1,2-Pentandiol oder 1,2-Hexandiol; Kollagen; Elastin oder Hyaluronsäure; Diacyladipate; Petrolatum; Urocaninsäure; Lecithin; Panthenol; Phytantriol; Lycopen; (Pseudo-)Ceramide; Glykosphingolipide; Cholesterol; Phytosterole; Chitosan; Chondroitinsulfat; Lanolin; Lanolinester; Aminosäuren; alpha-Hydroxysäuren (z. B. Zitronensäure, Milchsäure, Äpfelsäure) und deren Derivate; Mono-, Di- und Oligosaccharide, bevorzugt Glucose, Galactose, Fructose, Mannose, Fruchtzucker und Lactose; Polyzucker, bevorzugt β-Glucane, insbesondere 1,3-1,4-β-Glucan aus Hafer; alpha-Hydroxy-Fettsäuren; Triterpensäuren, bevorzugt Betulinsäure oder Ursolsäure; Algenextrakte.

Die Einsatzkonzentration der Feuchthalteregulatoren liegt je nach Substanz im Konzentrationsbereich von 0,1 bis 10 Gew.-% und bevorzugt im Konzentrationsbereich von 0,5 - 5 Gew.-%, jeweils bezogen auf die Gesamtmasse eines anwendungsbereiten kosmetischen oder pharmazeutischen Endprodukts.

### Kombination mit Kühlwirkstoffen:

Ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, lassen sich besonders vorteilhaft zusätzlich mit ein, zwei, drei oder mehreren Kühlwirkstoffen kombinieren. Einzelne zur Verwendung im Rahmen der vorliegenden Erfindung bevorzugte Kühlwirkstoffe sind nachfolgend aufgelistet. Der Fachmann kann die nachfolgende Liste um eine Vielzahl weiterer Kühlwirkstoffe ergänzen; die aufgelisteten Kühlwirkstoffe können auch in Kombination miteinander eingesetzt werden: I-Menthol, d-Menthol, racemisches Menthol, Menthonglycerinacetal (Handelsname: Frescolat^{®}MGA), Menthyllactat (Handelsname: Frescolat^{®}ML, vorzugsweise handelt es sich bei Menthyllactat um I-Menthyllactat, insbesondere I-Menthyl-I-lactat), substituierte Menthyl-3-carbonsäureamide (z.B. Menthyl-3-carbonsäure-N-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, N-Acetylglycinmenthylester, Isopulegol, Menthylhydroxycarbonsäureester (z.B. Menthyl-3-hydroxybutyrat), Monomenthylsuccinat, 2-Mercaptocyclodecanon, Menthyl-2-pyrrolidin-5-oncarboxylat, 2,3-Dihydroxy-p-menthan, 3,3,5-trimethylcyclohexanonglycerinketal, 3-Menthyl-3,6-di- und -trioxaalkanoate, 3-Menthyl methoxyacetat, Icilin.

Bevorzugte Kühlwirkstoffe sind: I-Menthol, d-Menthol, racemisches Menthol, Menthonglycerinacetal (Handelsname: Frescolat^{®}MGA), Menthyllactat (vorzugsweise I-Menthyllactat, insbesondere I-Menthyl-I-lactat, Handelsname: Frescolat^{®}ML), substituierte Menthyl-3-carbonsäureamide (z.B. Menthyl-3-carbonsäureN-ethylamid), 2-Isopropyl-N-2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, Isopulegol.

Besonders bevorzugte Kühlwirkstoffe sind: I-Menthol, racemisches Menthol, Menthonglycerinacetal (Handelsname: Frescolat^{®}MGA), Menthyllactat (vorzugsweise I-Menthyllactat, insbesondere I-Menthyl-I-lactat, Handelsname: Frescolat^{®}ML), 3-Menthoxypropan-1,2-diol, 2-Hydroxyethylmenthylcarbonat, 2-Hydroxypropylmenthylcarbonat.

Ganz besonders bevorzugte Kühlwirkstoffe sind: I-Menthol, Menthonglycerinacetal (Handelsname: Frescolat^{®}MGA), Menthyllactat (vorzugsweise I-Menthyllactat, insbesondere I-Menthyl-I-lactat, Handelsname: Frescolat^{®}ML).

Die Einsatzkonzentration der einzusetzenden Kühlwirkstoffe liegt je nach Substanz vorzugsweise im Konzentrationsbereich von 0,01 bis 20 Gewichtsprozent und bevorzugt im Konzentrationsbereich von 0,1 bis 5 Gewichtsprozent, bezogen auf die Gesamtmasse eines anwendungsbereiten kosmetischen oder pharmazeutischen Endprodukts.

### Kombination mit Osmolyten:

Weiterhin können ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol zusammen mit Osmolyten eingesetzt werden. Als Osmolyte seien beispielhaft genannt: Substanzen aus der Gruppe der Zuckeralkohole (myo-Inositol, Mannitol, Sorbitol), quaternäre Amine wie Taurin, Cholin, Betain, Betain-Glycin, Ectoin, Diglycerolphosphat, Phosphorylcholin, Glycerophosphorylcholine, Aminosäuren wie Glutamin, Glycin, Alanin, Glutamat, Aspartat oder Prolin, Phosphatidylcholin, Phosphatidylinositol, anorganische Phosphate, sowie Polymere der genannten Verbindungen wie Proteine, Peptide, Polyaminosäuren und Polyole. Alle Osmolyte haben zugleich eine hautbefeuchtende Wirkung.

### Kombination mit pflegenden Substanzen:

Zu pflegenden Substanzen, die sich mit ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, kombinieren lassen, zählen tierische und/oder pflanzliche Fette und Öle wie Olivenöl, Sonnenblumenöl, raffiniertes Sojaöl, Palmöl, Sesamöl, Rapsöl, Mandelöl, Borretschöl, Nachtkerzenöl, Kokosöl, Sheabutter, Jojobaöl, Spermöl, Rindertalg, Klauenöl und Schweineschmalz sowie gegebenfalls weitere pflegende Bestandteile wie zum Beispiel Fettalkohole mit 8-30 C-Atomen. Die verwendeten Fettalkohole können hierbei gesättigt oder ungesättigt bzw. linear oder verzweigt sein.

Zu pflegenden Substanzen, die sich besonders bevorzugt mit den erfindungsgemäßen C10-C14-Alkan-1,2-diolen kombinieren lassen, zählen darüber hinaus insbesondere auch
- Ceramide, wobei man unter Ceramiden N-Acylsphingosine (Fettsäureamide des Sphingosins) oder synthetische Analoga solcher Lipide (sogenannte Pseudo-Ceramide) versteht, die das Wasserhaltevermögen des Stratum Corneums deutlich verbessern.
- Phospholipide, beispielsweise Sojalecithin, Ei-Lecithin und Kephaline
- Vaseline, Paraffin- und Silikonöle; zu letzteren zählen unter anderem Dialkyl-und Alkylarylsiloxane wie Dimethylpolysiloxan und Methylphenylpolysiloxan, sowie deren alkoxylierte und quaternierte Derivate

### Kombination mit Konservierungsmitteln bzw. Chelatoren:

Kosmetische Zubereitungen, die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, enthalten, können auch ein, zwei, drei oder mehrere Wirkstoffe zur Konservierung kosmetischer Produkte sowie schweißhemmende Wirkstoffe (Antitranspirantien) und (Metall)-Chelatoren enthalten.

Vorzugsweise werden hierbei ein, zwei, drei oder mehrere Konservierungsmittel ausgewählt aus der Gruppe bestehend aus Benzoesäure und ihre Ester sowie Salze, Propionsäure und ihre Ester sowie Salze; Salicylsäure und ihre Ester sowie Salze, 2,4-Hexadiensäure (Sorbinsäure) und ihre Ester sowie Salze; Formaldehyd und Paraformaldehyd, 2-Hydroxybiphenylether und seine Salze, 2-Zinksulfidopyridin-N-oxid, anorganische Sulfite und Bisulfite, Natriumjodat, Chlorbutanolum, 4-Ethylquecksilber-(II)5-Amino-1,3-bis(2-Hydroxybenzoesäure, ihre Salze sowie Ester, Dehydratcetsäure, Ameisensäure, 1,6-Bis(4-amidino-2-bromphenoxy)-n-hexan und seine Salze, das Natriumsalz der Ethylquecksilber-(II)-thiosalicylsäure, Phenylquecksilber und seine Salze, 10-Undecylensäure und ihre Salze, 5-Amino-1,3-bis(2-ethylhexyl)-5-methyl-hexahydropyrimidin, 5-Brom-5-nitro-1,3-dioxan, 2-Brom-2-nitro-1,3-propandiol, 2,4-Dichlorbenzylalkohol, N-(4-Chlorphenyl)-N'-(3,4-dichlorphenyl)-harnstoff, 4-Chlor-m-kresol, 2,4,4'-Trichlor-2'-hydroxy-diphenylether, 4-Chlor-3,5-dimethylphenol, 1,1'-Methylen-bis(3-(1-hydroxymethyl-2,4-dioximidazolidin-5- yl)harnstoff), Poly-(hexamethylendiguanid)-hydrochlorid, 2-Phenoxyethanol, Hexamethylentetramin, 1-(3-Chloroallyl)-3,5,7-triaza-1-azoniaadamantanchlorid, 1(4-Chlorphenoxy)1(1H-imidazol-1-yl)-3,3-dimethyl-2-butanon, 1,3-Bis-(hydroxy-methyl)-5,5-dimethyl-2,4-imidazolidindion, Benzylalkohol, Octopirox, 1,2-Dibrom-2,4-dicyanobutan, 2,2'-Methylen-bis(6-brom-4-chlorphenol), Bromchlorophen, Mischung von 5-Chlor-2-methyl-3(2H)-isothiazolinon und 2-Methyl-3(2H)isothiazlinon mit Magnesiumchlorid und Magnesiumnitrat, 2-Benzyl-4-chlorphenol, 2-Chloracetamid, Chlorhexidin, Chlorhexidinacetat, Chlorhexidingluconat, Chlorhexidinhydrochlorid, 1-Phenoxy-propan-2-ol, N-Alkyl(C₁₂-C₂₂)trimethylammoniumbromid und -chlorid, 4,4-Dimethyl-1,3-oxazo-lidin, N-Hydroxymethyl-N-(1,3-di(hydroxymethyl)-2,5-dioxoimidazolidin-4-yl)-N'-hydroxy-methylharnstoff, 1,6-Bis(4-amidino-phenoxy)-n-hexan und seine Salze, Glutaraldehyd, 5-Ethyl-1-aza-3,7-dioxabicyclo(3.3.0)octan, 3-(4-Chlorphenoxy)-1,2-propandiol, Hyamine, Alkyl-(C₈-C₁₈)-dimethyl-benzyl-ammoniumchlorid, Alkyl-(C₈-C₁₈)-dimethyl-benzylammonium-bromid, Alkyl-(C₈-C₁₈)-dimethyl-benzyl-ammoniumsaccharinat, Benzylhemiformal, 3-Jod-2-propinyl-butylcarbamat, Natrium-hydroxymethyl-aminoacetat oder Natrium-hydroxymethyl-aminoacetat.

Bevorzugt einzusetzende (Metall)-Chelatoren sind hierbei u.a α-Hydroxyfettsäuren, Phytinsäure, Lactoferrin, α-Hydroxysäuren und ihre Salze, bevorzugt Zitronensäure oder Äpfelsäure, sowie Galactarsäure, Galacturonsäure, Gluconsäure, Glucuronsäure, Ribonsäure und deren Salze, Huminsäuren, Gallensäuren, Gallenextrakte, Bilirubin, Biliverdin bzw. EDTA, EGTA und deren Derivate.

### Kombination mit tierischen und/oder pflanzlichen Proteinhydrolysaten:

Ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, können vorteilhaft auch tierische und/oder pflanzliche Proteinhydrolysate zugesetzt werden. Vorteilhaft sind insoweit insbesondere Elastin-, Kollagen-, Keratin-, Milcheiweiß-, Sojaprotein-, Haferprotein-, Erbsenprotein-, Mandelprotein-, und Weizenproteinfraktionen oder entsprechende Proteinhydrolysate aber auch deren Kondensationsprodukte mit Fettsäuren sowie quarternisierte Proteinhydrolysate, wobei die Verwendung pflanzlicher Proteinhydrolysate bevozugt ist.

### Kombination mit Lösungsmitteln:

Sofern eine kosmetische und/oder dermatologische, ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol enthaltende Zubereitung eine Lösung oder Lotion darstellt, können als Lösungsmittel verwendet werden:
- Wasser oder wässrige Lösungen;
- fette Öle, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, - monoethyl- oder -monobutylether, Diethylenglykolmonomethyl- oder - monoethylether und analoge Produkte.

Insbesondere werden Gemische der vorstehend genannten Lösungsmittel verwendet. Bei alkoholischen Lösungsmitteln kann Wasser ein weiterer Bestandteil sein.

### Kombination mit Antioxidantien:

Kosmetische Zubereitungen, die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, enthalten, können auch ein, zwei, drei oder mehrere Antioxidantien enthalten, wobei alle für kosmetische und/oder dermatologische Anwendungen geeigneten oder gebräuchlichen Antioxidantien verwendet werden können.

### Kombination mit Vitaminen:

Kosmetische Zubereitungen, die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, enthalten, können auch ein, zwei, drei oder mehrere Vitamine und/oder Vitaminvorstufen enthalten, wobei alle für kosmetische und/oder dermatologische Anwendungen geeigneten oder gebräuchlichen Vitamine oder Vitaminvorstufen verwendet werden können.

### Kombination mit Hautaufhellern:

Ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, können in zahlreichen Fällen vorteilhaft in Kombination mit ein, zwei, drei oder mehreren hautaufhellenden Wirkstoffen eingesetzt werden. Erfindungsgemäß können hierbei alle für kosmetische und/oder dermatologische Anwendungen geeigneten oder gebräuchlichen hautaufhellenden Wirkstoffe verwendet werden. Vorteilhafte hautaufhellende Wirkstoffe sind insoweit Kojisäure (5-Hydroxy-2-hydroxymethyl-4-pyranon), Kojisäurederivate wie z.B. Kojisäuredipalmitat, Arbutin, Ascorbinsäure, Ascorbinsäurederivate, Hydrochinon, Hydrochinonderivate, Resorcin, schwefelhaltige Moleküle wie z.B. Glutathion oder Cystein, alpha-Hydroxysäuren, (z.B. Zitronensäure, Milchsäure, Apfelsäure) und deren Derivate, N-Acetyl-Tyrosin und -Derivate, Undecenoylphenylalanin, Gluconsäure, 4-Alkylresorcine, Diphenylmethan-Derivate wie z.B. 4-(1-Phenylethyl)1,3-benzenediol Chromon-Derivate wie Aloesin, Flavonoide, Thymolderivate, 1-Aminoethylphosphinsäure, Thioharnstoffderivate, Ellagsäure, Nicotinamid, Zinksalze wie z.B. Zinkchlorid oder -gluconat, Thujaplicin und -Derivate, Triterpene wie Maslinsäure, Sterole wie Ergosterol, Benzofuranone wie Senkyunolid, Vinyl- und Ethylgujacol, Inhibitoren der Stickstoffoxid-Synthese wie z.B., L-Nitroarginin und dessen Derivate, 2,7-Dinitroindazol oder Thiocitrullin, Metallchelatoren (z.B. α-Hydroxyfettsäuren, Palmitinsäure, Phytinsäure, Lactoferrin, Huminsäure, Gallensäure, Gallenextrakte, Bilirubin, Biliverdin, EDTA, EGTA und deren Derivate), Retinoide, Sojamilch, Serin-Protease-Inhibitoren oder Liponsäure oder andere synthetische oder natürliche Wirkstoffe zur Haut- und Haaraufhellung, wobei letztere auch in Form eines Extrakts aus Pflanzen, wie z.B. Bearberry-Extrakt, Reis-Extrakt, Süßholzwurzel-Extrakt oder daraus angereicherte Bestandteile wie Glabridin oder Licochalkon A, Artocarpus-Extrakt, Extrakt von Rumex- und Ramulus-Arten, Extrakte aus Kieferarten (Pinus) und Extrakte aus Vitis-Arten oder daraus angereicherte Stilbenderivate, Extrakt aus Saxifraga, Maulbeer, Scutelleria oder/und Trauben verwendet werden.

### Kombination mit Hautbräunern:

Kosmetische Zubereitungen, die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, enthalten, können auch ein, zwei, drei oder mehrere Wirkstoffe mit hautbräunender Wirkung enthalten. Es können insoweit alle für kosmetische und/oder dermatologische Anwendungen geeigneten oder gebräuchlichen hautbräunenden Wirkstoffe verwendet werden. Beispielhaft sei hier das Dihydroxyaceton (DHA; 1,3-Dihydroxy-2-propanon) erwähnt. DHA kann sowohl in monomerer als auch in dimerer Form vorliegen, wobei in kristalliner Form der Anteil an Dimeren überwiegt.

### Kombination mit Sacchariden:

Kosmetische Zubereitungen, die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, enthalten, können auch ein, zwei, drei oder mehrere Mono- Di- und Oligosaccharide, bevorzugt Glucose, Galactose, Fructose, Mannose, Fruchtzucker und Lactose enthalten.

### Kombination mit Pflanzenextrakten:

Kosmetische Zubereitungen, die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, enthalten, können auch ein, zwei, drei oder mehrere Pflanzenextrakte enthalten, die üblicherweise durch Extraktion der gesamten Pflanze, in einzelnen Fällen aber auch ausschließlich aus Blüten und/oder Blättern, Holz, Rinde oder Wurzeln der Pflanze hergestellt werden.

### Kombination mit Tensiden:

Kosmetische Zubereitungen, die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, enthalten, können, insbesondere wenn kristalline oder mikrokristalline Festkörper, beispielsweise anorganische Mikropigmente in die Zubereitungen eingearbeitet werden sollen, auch ein, zwei, drei oder mehrere anionische, kationische, nichtionische und/oder amphotere Tenside enthalten. Tenside sind amphiphile Stoffe, die organische, unpolare Substanzen in Wasser lösen können. Bei den hydrophilen Anteilen eines Tensidmoleküls handelt es sich dabei meist um polare funktionelle Gruppen, beispielweise -COO-, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch. Typisch für nicht-ionische Tenside sind Polyether-Ketten. Nicht-ionische Tenside bilden in wässrigem Medium keine Ionen.

### A. Anionische Tenside

Vorteilhaft zu verwendende anionische Tenside sind Acylaminosäuren (und deren Salze), wie
- Acylglutamate, beispielsweise Natriumacylglutamat, Di-TEA-palmitoylaspartat und Natrium Capryl/ Capringlutamat,
- Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
- Sarcosinate, beispielsweise Myristoyl Sarcosin, TEA-Iauroylsarcosinat, Natriumlauroylsarcosinat und Natriumcocoylsarkosinat,
- Taurate, beispielsweise Natriumlauroyltaurat und Natriummethylcocoyltaurat,
- Acyllactylate, Lauroyllactylat, Caproyllactylat, Stearoyllactylat
- Alaninate

Carbonsäuren und Derivate, wie beispielsweise Laurinsäure, Aluminiumstearat, Magnesiumalkanolat und Zinkundecylenat,
- Ester-Carbonsäuren, beispielsweise Calciumstearoyllactylat, Laureth-6 Citrat und Natrium PEG-4 Lauramidcarboxylat,
- Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,

Sulfonsäuren und Salze, wie
- Acyl-isothionate, z.B. Natrium-/ Ammoniumcocoyl-isethionat,
- Alkylarylsulfonate,
- Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
- Sulfosuccinate, beispielsweise Dioctylnatriumsulfosuccinat, Dinatriumlaureth-sulfosuccinat, Dinatriumlaurylsulfosuccinat und Dinatriumundecylenamido MEA-Sulfosuccinat
sowie
Schwefelsäureester, wie
- Alkylethersulfat, beispielsweise Natrium-, Ammonium-, Magnesium-, MIPA, TIPA-Laurethsulfat, Natriummyrethsulfat und Natrium C12-13 Parethsulfat,
- Alkylsulfate, beispielsweise Natrium-, Ammonium- und TEA- Laurylsulfat.

### B. Kationische Tenside

Vorteilhaft zu verwendende kationische Tenside sind
- Alkylamine,
- Alkylimidazole,
- Ethoxylierte Amine und
- Quaternäre Tenside.
   RNH₂CH₂CH₂COO⁻ (bei pH=7)
   RNHCH₂CH₂COO- B⁺ (bei pH=12) B⁺ = beliebiges Kation, z.B. Na⁺
- Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkylamidopropylhydroxysulfain. Die verwendeten kationischen Tenside können ferner bevorzugt gewählt werden aus der Gruppe der quaternären Ammoniumverbindungen, insbesondere Benzyltrialkyl-ammoniumchloride oder -bromide, wie beispielsweise Benzyldimethylstearyl-ammoniumchlorid, ferner Alkyltrialkylammoniumsalze, beispielsweise Cetyltrimethylammoniumchlorid oder -bromid, Alkyldimethylhydroxy-ethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
- Acyl-/dialkylethylendiamin, beispielsweise Natriumacylamphoacetat, Dinatrium-acylamphodipropionat, Dinatriumalkylamphodiacetat, Natriumacylamphohydroxy-propylsulfonat, Dinatriumacylamphodiacetat und Natriumacylamphopropionat,
- N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
- Alkohole,
- Alkanolamide, wie Cocamide MEA/ DEA/ MIPA,
- Aminoxide, wie Cocoamidopropylaminoxid,
- Ester, die durch Veresterung von Carbonsäuren mit Ethylenoxid, Glycerin, Sorbitan oder anderen Alkoholen entstehen,
- Ether, beispielsweise ethoxylierte/propoxylierte Alkohole, ethoxylierte/propoxylierte Ester, ethoxylierte/ propoxylierte Glycerinester, ethoxylierte/propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid undCocoglycosid.
- Sucroseester, -Ether
- Polyglycerinester, Diglycerinester, Monoglycerinester
- Methylglucoseester, Ester von Hydroxysäuren

Vorteilhaft ist ferner die Verwendung einer Kombination von anionischen und/oder amphoteren Tensiden mit einem oder mehreren nicht-ionischen Tensiden.

Die oberflächenaktive Substanz kann in einer Konzentration zwischen 1 und 98 Gew.-% in den erfindungsgemäße Alkan-1,2-diole mit einer Kettenlänge von C10 bis C14 enthaltenden Zubereitungen vorliegen, bezogen auf das Gesamtgewicht der Zubereitungen.

### Emulsionen umfassend eine erfindungsgemäße Mischung:

Kosmetische oder dermatologische Zubereitungen, die erfindungsgemäße Alkan-1,2-diole mit einer Kettenlänge von C10 bis C14 enthalten, können auch als Emulsionen vorliegen.

Die Ölphase kann vorteilhaft gewählt werden aus folgender Substanzgruppe:
- Mineralöle, Mineralwachse
- fette Öle, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Vorteilhaft einsetzbar sind nicht komedogen aktive Ester aus gesättigten und/oder ungesättigten verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen. Bevorzugte Esteröle sind 3,5,5-Trimethylhexyl-3,5,5-trimethylhexanoat, 2-Ethylhexylisononanoat, 2-Ethylhexyl-3,5,5-trimethylhexanoat, 2-Ethylhexyl-2-ethylhexanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl vorteilhaft mit den erfindungsgemäßen Alkan-1,2-diolen mit einer Kettenlänge von C10 bis C14 kombiniert werden.

Daneben können aber auch Ester aus aromatischen Carbonsäuren und gesättigten und/oder ungesättigten, verzweigten und/oder unverzweigten Alkoholen einer Kettenlänge von 3 bis 30 C-Atomen.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr. Auch beliebige Abmischungen solcher Öl- und Wachskomponenten sind vorteilhaft einsetzbar. In manchen Fällen ist es auch vorteilhaft, Wachse, beispielsweise Cetylpalmitat, als alleinige Lipidkomponente der Ölphase einzusetzen, vorteilhaft wird die Ölphase gewählt aus der Gruppe, die besteht aus 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäuretriglycerid und Dicaprylylether. Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat. Auch die Kohlenwasserstoffe Paraffinöl, Squalan und Squalen lassen sich vorteilhaft verwenden. Vorteilhaft kann die Ölphase ferner einen Gehalt an cyclischen oder linearen Silikonölen aufweisen oder vollständig aus solchen Ölen bestehen, wobei es allerdings bevorzugt ist, außer dem Silikonöl oder den Silikonölen einen zusätzlichen Gehalt an anderen Ölphasenkomponenten zu verwenden. Cyclomethicon (z.B. Decamethylcyclopentasiloxan) kann vorteilhaft als Silikonöl eingesetzt werden. Aber auch andere Silikonöle sind vorteilhaft verwendbar, beispielsweise Undecamethylcyclotrisiloxan, Polydimethylsiloxan und Poly(methyl-phenylsiloxan). Besonders vorteilhaft sind ferner Mischungen aus Cyclomethicon und Isotridecylisononanoat sowie aus Cyclomethicon und 2-Ethylhexylisostearat.

Die wässrige Phase von als Emulsion vorliegenden Zubereitungen, die erfindungsgemäße Alkan-1,2-diole mit einer Kettenlänge von C10 bis C14 enthalten, kann vorteilhafterweise umfassen: Alkohole, Diole oder Polyole niedriger C-Zahl, sowie deren Ether, vorzugsweise Ethanol, Isopropanol, Propylenglykol, Glycerin, Ethylenglykol, Ethylenglykol- monoethyl - o der -monobutylether, Propylenglykolmonomethyl, -monoethyl- oder -monobutylether, Diethylenglykolmonomethyl-oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropyl-methylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Als Emulsion vorliegende Zubereitungen, die erfindungsgemäße Alkan-1,2-diole mit einer Kettenlänge von C10 bis C14 enthalten, umfassen vorteilhaft einen oder mehrere Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-COO-(-CH₂-CH₂-O-)ₙ-OOH,

   und n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel R-O-(-CH₂-CH(CH₃)-O-)ₙ-H
- der Polypropylenglycolether der allgemeinen Formel

   R-O-(-CH₂-CH(CH₃)-O-)ₙ-R'
- der propoxylierten Wollwachsalkohole,
- der veretherten Fettsäurepropoxylate R-COO-(-CH₂-CH(CH₃)-O-)ₙ-R'
- der veresterten Fettsäurepropoxylate der allgemeinen Formel

   R-COO-(-CH₂-CH(CH₃)-O-)ₙ-C(O)-R'
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)-O-)ₙ-CH₂-COOH,
- der Alkylethersulfate bzw. die diesen Sulfaten zugrundeliegenden Säuren

   der allgemeinen Formel R-O-(-CH₂-CH(CH₃)-O-)ₙ-SO₃-H,
- der Fettalkoholethoxylate/propoxylate der allgemeinen Formel R-O-Xₙ-Yₘ-H
- der Polypropylenglycolether der allgemeinen Formel R-O-Xₙ-Yₘ-R'
- der veretherten Fettsäurepropoxylate der allgemeinen Formel R-COO-Xₙ-Yₘ-R'
- der Fettsäureethoxylate/propoxylate der allgemeinen Formel R-COO-Xₙ-Yₘ-H.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19), Polyethylenglycol(20)isostearyl-ether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether(Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(1 4)oleylether (Oleth-14), Polyethylenglycol(15)oleylether (Oleth-15), Polyethylenglycol(12)laurylether (Laureth-12), Polyethylenglycol(12)isolaurylether (Isolaureth12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20).

Es ist ferner von Vorteil, die Fettsäureethoxylate aus folgender Gruppe zu wählen:

Polyethylenglycol(20)stearat, Polyethylenglycol(21)stearat, Polyethylenglycol-(22)stearat, Polyethylenglycol(23)stearat, Polyethylenglycol(24)stearat, Polyethylenglycol(25)stearat, Polyethylenglycol(12)isostearat, Polyethylenglycol-(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat, Polyethylenglycol(12)oleat, Polyethylenglycol(13)-oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth 1-4 sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprylat/caprinat, Polyethylenglycol(20)glyceryloleat, Polyethylenglycol(20)glycerylisostearat, Polyethylenglycol(1 8)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis 18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycolester gesättigter und/oder ungesättigter, verzweigter und/oder unverzweigter Alkancarbonsäuren einer Kettenlänge von 8 bis 24, insbesondere 12 bis 18 C-Atomen sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether(Steareth-2), Glycerylmonolaurat, Glycerylmonocaprinat, Glycerylmonocaprylat.

### Bevorzugte Formulierungen:

### Kombination mit Sonnenschutzmitteln:

Zur Anwendung werden die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol enthaltenden kosmetischen und/oder dermatologischen/keratologischen Formulierungen in der für Kosmetika und Dermatika üblichen Weise auf die Haut und/oder die Haare in ausreichender Menge aufgebracht. Besondere Vorteile bieten dabei auch kosmetische und/oder dermatologische Zubereitungen, die ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol enthalten und zusätzlich ein, zwei, drei oder mehrere Verbindungen, die als Sonnenschutzmittel wirken. Vorteilhaft enthalten diese Zubereitungen mindestens einen UVA-Filter und/oder mindestens einen UVB-Filter und/oder mindestens ein anorganisches Pigment. Die Zubereitungen können dabei in verschiedenen Formen vorliegen, wie sie z. B. üblicherweise für diesen Typ von Zubereitungen eingesetzt werden. So können sie bevorzugt eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, können vorteilhaft auch mit ein, zwei, drei oder mehreren Verdickern kombiniert werden. Geeignete Verdicker werden ausgewählt aus der Gruppe bestehend aus Homopolymere der Acrylsäure mit einem Molekulargewicht von 2000000 bis 6000000, bevorzugt Handelsprodukt Carbopole. Weitere bevorzugte Verdicker werden vertrieben unter den Namen Carbopol 940, Carbopol EDTA 2001 oder Modarez V 600 PX. Polymere aus Acrylsäure und Acrylamid (Natriumsalz) mit einem Molekulargewicht von 2000000 bis 6000000 wie z.B. Hostacerin PN 73 oder das unter dem Namen Amigel vertriebene Sclerotium Gum. Ausserdem geeignet sind Copolymere der Acrylsäure oder der Methacrylsäure wie z.B. Carbopol 1342 oder Permulen TRI.

Weitere Verdickertypen sind Polyglycole, Cellulosederivate, insbesondere Hydroxyalkylcellulosen sowie Alginate, Carageenan und anorganische Verdicker wie z.B. natürliche oder synthetische Bentonite.

### Kombination mit kosmetischen Hilfsstoffen:

Ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol können in kosmetischen Zubereitungen vorteilhaft auch mit ein, zwei, drei oder mehreren kosmetischen Hilfsstoffen kombiniert werden, wie sie üblicherweise in solchen Zubereitungen verwendet werden, bevorzugt Antioxidantien, Parfümöle, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, weitere anfeuchtende und/oder feuchthaltende Substanzen, Fette, Öle, Wachse oder andere übliche Bestandteile einer kosmetischen Formulierung, wie bevorzugt Alkohole, Polyole, Polymere, Schaumstabilisatoren, Elektrolyte, organische Lösungsmittel oder Silikonderivate. Erfindungsgemäß können hierbei alle denkbaren für kosmetische und/oder dermatologische Anwendungen geeigneten oder gebräuchlichen Antioxidantien, Parfümöle, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse, Alkohole, Polyole, Polymere, Schaumstabilisatoren, Elektrolyte, organische Lösungsmittel oder Silikonderivate verwendet werden.

### Kombination mit Riechstoffen:

Ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol können auch als Bestandteil von Duftstoffkompositionen (Riechstoffkompositionen) für Haar- und/oder Kopfhautpflegeprodukte eingesetzt werden und insbesondere aufgrund ihrer spezifischen Wirksamkeit beispielsweise einem parfümierten Fertigprodukt eine zusätzliche Antischuppen-Wirkung verleihen. Besonders bevorzugte Duftstoffkompositionen umfassen oder bestehen aus (a) einer sensorisch wirksamen Menge eines Riechstoffe oder der Summe aus zwei, drei oder mehreren Riechstoffen, (b) einer Schuppenreduzierende Menge eines oder mehrerer, bevorzugt eines, zweier oder dreier erfindungsgemäßer C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol sowie (c) gegebenenfalls ein oder mehreren Träger- und/oder Zusatzstoffen.

Da der Anteil an Duftstoffkomposition (Parfüm) in einem kosmetischen Fertigprodukt häufig im Bereich von ca. 1 Gew.-% liegt, enthält eine Duftstoffkomposition vorzugsweise 0,1-10 Gew.-% ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, jeweils bezogen auf das Gesamtgewicht der Duftstoffkomposition.

Als besonders vorteilhaft hat es sich erwiesen, dass die erfindungsgemäßen C10-C14-Alkan-1,2-diole jeweils nur einen schwachen Eigengeruch besitzen oder gar völlig geruchlos sind; denn diese Eigenschaft prädestiniert sie insbesondere für den Einsatz in einer Duftstoffkomposition.

Riechstoffe, die zur Kombination vorteilhafterweise geeignet sind, finden sich z.B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001. Im einzelnen seien als bevorzugt genannt:

**Extrakte aus natürlichen Rohstoffen** wie Etherische Öle, Concretes, Absolues, Resine, Resinoide, Balsame, Tinkturen wie z. B.:
Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
**Einzel-Riechstoffe** aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)-und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon ; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal ; acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Bevorzugte Trägerstoffe sind Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat und Triacetin.

Ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, lassen sich dabei ohne Schwierigkeiten in gängige kosmetische oder dermatologische/keratologische Formulierungen wie u.a. Pumpsprays, Aerosolsprays, Cremes, Shampoos, Salben, Tinkturen, Lotionen, und dergleichen einarbeiten. Hierbei ist es auch möglich und in manchen Fällen vorteilhaft, ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol mit weiteren Wirkstoffen zu kombinieren. Ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, enthaltenden kosmetischen und/oder dermatologischen/keratologischen Formulierungen können hierbei ansonsten wie üblich zusammengesetzt sein und zur Behandlung der Haut und/oder der Haare im Sinne einer dermatologischen/keratologischen Behandlung oder einer Behandlung im Sinne der pflegenden Kosmetik dienen.

Bevorzugte Ausgestaltungen der erfindungsgemäßen Mischungen sowie der erfindungsgemäßen Verfahren und Verwendungen lassen sich den nachfolgenden Beispielen und den zugehörigen Tabellen entnehmen:

### Ausführungsbeispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1

### Untersuchungen zur antimikrobiellen Wirkung von Alkan-1,2-diolen mit einer Kettenlänge von C5 bis C18 gegenüber Malassezia furfur (Synonym: Pityrosporum ovale);

### Allgemeine Testbedingungen (MHK-Wert-Messungen):

Der Nachweis der antimikrobiellen Wirkung der Alkan-1,2-diole gegenüber Malassezia furfur erfolgte mit Hilfe des Agardilutionsverfahren in Anlehnung an DIN 58 940/ICS und DIN 58 944/ICS. Als Testkeim wurde der Malassezia furfur Stamm DSM6171 verwendet.

Es wurden Petrischalen von 9 cm Durchmesser mit 8,7 ml frisch hergestelltem und bei 50 °C flüssig gehaltenem Mueller-Hinton-Agar (Merck, Art. 1.05437) verwendet der 3% Tween 80 (Merck, Art. 8.2217) enthielt. Zur Herstellung der Testkonzentrationen wurden ethanolische Stammlösungen der Alkan-1,2-Diol-Proben mit Ethanol 96% soweit verdünnt bis folgende endgültige Testkonzentrationen der Alkan-1,2-diole im Mueller-Hinton-Agar vorlagen: 25000ppm, 12500ppm, 6300ppm, 3600 ppm, 1800ppm, 1100ppm, 900ppm, 450ppm, 225ppm, 112ppm, 56ppm, 28ppm, 14ppm, 7ppm, 4ppm. Pro Testkonzentration und Nährmedium wurden 2 Agarplatten gegossen.

Nach Verfestigung und Trocknung (ca. 1 h bei 37 °C) wurden die Testplatten punktförmig mit jeweils 1 µl der Malassezia furfur (DSM 6171 - 2,8x10⁷ KBE/ml) Testkeimsuspensionen beimpft. Im Anschluß wurden die inokulierten Platten 72h bei 30 °C bebrütet und anschließend ausgewertet. Als MHK (Minimale Hemmkonzentration) wurde die niedrigste Wirkstoffkonzentration angesehen, bei der makroskopisch kein Wachstum vorhanden ist.

Gemäß den allgemeinen Testbedingungen wurden die MHK-Werte der in Tabelle 1 aufgelisteten Alkan-1,2-diole bestimmt.

**Tabelle 1:**

| **Testverbindung** | **MHK-Wert (ppm)** |
|---|---|
| 1,2 Pentandiol | 25000 |
| 1,2-Hexandiol | 12500 |
| 1,2-Octandiol | 225 |
| 1,2-Decandiol | 28 |
| 1,2-Dodecandiol | 14 |
| 1,2-Tetradecandiol | 7 |
| 1,2-Hexadecandiol | 1100 |
| 1,2-Octadecandiol | >1100 |

Wie die Untersuchungen zur antimikrobiellen Wirksamkeit von Alkan-1,2-diolen zeigen, besitzen 1,2-Pentandiol (MHK-Wert: 25000 ppm) und 1,2-Hexandiol (MHK-Wert: 12500 ppm) eine sehr schwache antimikrobielle Wirksamkeit gegenüber *Malassezia furfur* (Synonym: *Pityrosporum ovale).* 1,2-Hexadecandiol (MHK-Wert: 1100 ppm) und 1,2-Octadecandiol (MHK-Wert: >1100 ppm) sind ebenfalls nur äußerst schwach wirksam. Die stärkste antimikrobielle Wirksamkeit gegenüber *Malassezia furfur* konnte für 1,2-Decandiol, 1,2-Dodecandiol und 1,2-Tetradecandiol nachgewiesen werden. So besitzt 1,2-Decandiol mit einem MHK-Wert von 28 ppm eine gegenüber 1,2-Octandiol (MHK-Wert: 225 ppm) um etwa den Faktor 8 stärkere Wirksamkeit. 1,2-Dodecandiol mit einem MHK-Wert von 14 ppm besitzt gegenüber 1,2-Octandiol (MHK-Wert: 225 ppm) eine um etwa den Faktor 16 stärkere Wirksamkeit und 1,2-Tetradecandiol mit einem MHK-Wert von <7ppm ppm besitzt gegenüber 1,2-Octandiol (MHK-Wert: 225 ppm) eine um mindestens den Faktor 32 stärkere Wirksamkeit. Die stärkste Wirksamkeit innerhalb der untersuchten Alkan-1,2-diole gegenüber *Malassezia furfur* (Synonym: *Pityrosporum ovale)* konnte für 1,2-Tetradecandiol gefunden werden, wobei 1,2-Decandiol und 1,2-Dodecandiol mit MHK-Werten deutlich <100ppm ebenfalls in die Kategorie der sehr gut gegen *Malassezia furfur* wirksamen Antischuppen-Mittel einzustufen sind.

### Beispiel 2

Beispielrezepturen für Zubereitungen zur Prophylaxe und/oder Behandlung von malassezia-induzierter Schuppenbildung umfassen ein oder mehrere, bevorzugt ein, zwei oder drei erfindungsgemäße C10-C14-Alkandiole, weiter bevorzugt eine Verbindung oder eine Mischung aus zwei oder drei Verbindungen aus der Gruppe bestehend aus 1,2-Decandiol, 1,2-Dodecandiol und 1,2-Tetradecandiol, sowie ein, zwei, drei, vier, fünf, sechs, sieben, acht, neun, zehn, elf oder zwölf weitere Antischuppen-Mittel ausgewählt aus der Gruppe bestehend aus Climbazol, Ketoconazol, Zinkpyrithion, Steinkohleteer, Schwefel, Selendisulfide, Octopirox (INCI: Piroctone Olamine), Cyclopiroxolamin, Undecylensäure und deren Metallllsalze, Ichthyol (sulphoniertes Schieferöl), Sensiva SC-50 (Etyhlhexylglycerin) und/oder Vetikol (4-Methyl-4-phenyl-2-pentanol).

| **ROHSTOFFNAME (Lieferant)** | **INCI** | **GEWICHTS %** | | | | | | | |
|---|---|---|---|---|---|---|---|---|---|
| | | **1** | **2** | **3** | **4** | **5** | **6** | **7** | **8** |
| Allantoin (Merck) | Allantoin | - | - | - | 0,1 | - | - | - | - |
| Aloe Vera Gel Concentrate 10/1 (Symrise) | Water (Aqua), Aloe Barbadensis Leaf Juice | 1,0 | - | - | 0,5 | - | - | - | - |
| AMP-95 (Angus) | Aminomethylpropanol | - | - | - | - | - | 0,7 | - | - |
| Antil 141 Liquid (Degussa-Goldschmidt) | Propylene Glycol, PEG-55 Propylene Glycol Distearate | - | 1,0 | - | - | - | - | - | - |
| Antil 200 (Degussa-Goldschmidt) | PEG-200 Hydrogenated Glyceryl Palmitate, PEG-7 Glyceryl Cocoate | 1,5 | - | - | - | - | - | - | - |
| Bisabolol, (-) alpha (Symrise) | Bisabolol | - | - | - | - | - | - | 0,2 | |
| Carbopol ETD 2001 (Noveon) | Carbomer | - | - | - | - | 0,7 | - | - | - |
| Cetiol OE (Cognis) | Dicaprylyl Ether | - | - | 7,2 | - | - | - | - | - |
| Dracorin GMS (Symrise) | Glyceryl Stearate | - | - | - | - | - | - | - | 4,0 |
| Cetiol S (Cognis) | Diethylhexylcyclohexane | - | - | 7,0 | - | - | - | - | - |
| Cremogen AlphaPulp (Symrise) | Water (Aqua),Butylene Glycol, - Malic Acid, Prunus Amygdalus Dulcis (Sweet Almond) Seed Extract, Actinidia Chinensis (Kiwi) Fruit Juice, Citrus Aurantium Dulcis (Orange) Juice, Citrus Paradisi (Grapefruit) Juice, Pyrus Malus (Apple) Juice, PEG-40 Hydrogenated Castor Oil | - | - | - | 1,0 | - | - | - | - |
| Crinipan AD (Symrise) | Climbazole | - | - | 0,3 | - | 0,5 | - | - | - |
| Cyclopiroxolamine | Cyclopiroxolamine | | | | | | | | |
| Decandiol, 1,2 | 1,2 Decandiol | 5,0 | - | - | 1,0 | - | 0,2 | 0,1 | 1,0 |
| Dehyquart A CA (Cognis) | Cetrimonium Chloride | - | 1,0 | 4,0 | - | - | - | - | - |
| Dehyton K (Cognis) | Cocoamidopropyl Betaine | 6,0 | 8,0 | - | - | - | - | - | - |
| Dihydroavenanthramid D (Symrise) | Dihydroavenanthramide D | 0,05 | - | - | - | 0,05 | - | - | 0,05 |
| Dodecandiol, 1,2 | | - | 1,0 | 0,5 | 2,0 | 0,1 | - | 0,1 | - |
| D-Panthenol 75L (DSM Nutritional) | Panthenol | - | 1,0 | 1,0 | - | - | - | - | - |
| Dracorin CE (Symrise) | Glyceryl Stearate Citrate | - | - | - | 0,3 | - | - | - | - |
| Dracorin GOC (Symrise) | Glyceryl Oleate Citrate | - | - | - | 0,5 | - | - | - | - |
| Dracorin 100 s.e. P (Symrise) | PEG-100 Stearate, Glyceryl Stearate | - | - | 1,0 | - | - | - | - | 6,0 |
| Drago-Beta-Glucan (Symrise) | Water, Butylene Glycol, Glycerin, Avena Sativa (Oat Kernel Extract) | 0,3 | 0,3 | - | - | - | - | - | - |
| Dragocare W | PEG-40 Butyloctanol Wheat | - | - | 0,5 | 1,0 | - | - | - | - |
| (Symrise) | Germ Esters, Water (Aqua), Lactic Acid, Tocopherol | | | | | | | | |
| Dragocide Liquid (Symrise) | Phenoxyethanol, Methyl,- Ethyl, Butyl, Propyl, Isobutyl-paraben | 0,8 | 0,8 | 0,8 | 0,8 | 0,5 | - | - | - |
| Dragoderm (Symrise) | Glycerin, Triticum Vulgare (Wheat) Gluten, Water (Aqua) | 0,3 | 1,0 | - | 2,0 | - | - | - | - |
| Dragoxat 89 (Symrise) | Ethyl Hexylisononanoate | - | - | 1,0 | - | - | - | 5,0 | - |
| Emulsiphos (Symrise) | Potassium Cetyl Phosphate, Hydrogenated Palm Glycerides | - | - | 2,0 | - | - | - | - | - |
| Ethanol 96 %ig | Ethanol | - | - | - | - | 30,0 | 30,0 | 0,3 | - |
| Eumulgin B1 (Cognis) | Ceteareth-12 | - | - | 3,5 | - | - | - | - | - |
| Euperlan PK 4000 (Cognis) | Glycol Distearate, Laureth-4, Cocoamidopropyl Betaine | 1,0 | 2,5 | - | - | - | - | - | - |
| Extrapone Champagne GW (Symrise) | Water (Aqua), Glycerin, Wine - Extract, Alcohol | - | - | 2,0 | - | - | - | - | - |
| Extrapone Passion Flower (Symrise) | Water (Aqua), Propylene Glycol, Passiflora Incarnata Extract, Glucose | 0,5 | - | - | - | - | - | - | - |
| Farnesol (Symrise) | Farnesol | - | - | - | - | - | 0,3 | 0,1 | - |
| Frescolat ML (Symrise) | Menthyl Lactate | - | - | - | - | 0,5 | 0,3 | 0,5 | - |
| Genapol LRO liquid (Clariant) | Sodium Laureth Sulfate | 35,0 | - | - | - | - | - | - | - |
| Glycerin, 99,5% | Glycerin | - | - | - | - | 10,0 - | | - | - |
| Hair Conditioner Base (Symrise) | Cetyl Alcohol, Behentrimonium Chloride, Triticum Vulgare (Wheat) Bran Extract, Linoleic Acid | - | - | - | 3,0 | - | - | - | - |
| Hydrolite-5 (1,2 Pentandiol) (Symrise) | Pentylene Glycol | - | - | 0,5 | 0,5 | 1,0 | - | - | - |
| Ichthyol | Sodium Shale Oil Sulfonate | 0,5 | 0,5 | - | - | - | - | - | - |
| Isoadipate (Symrise) | Diisopropyl Adipate | - | - | - | - | 0,5 | 0,3 | - | - |
| Isodragol (Symrise) | Triisononanoin | - | - | - | - | - | - | 1,0 | 3,0 |
| Ketoconazol | Ketoconazol | 0,1 | - | 0,5 | - | - | - | - | 0,5 |
| PCL Liquid 100 (Symrise) | Cetearyl Octanoate | - | - | - | - | - | 0,2 | - | 2,0 |
| Luviskol K 30 Powder (BASF AG) | PVP | - | - | - | - | 3,0 | - | - | - |
| Luviskol VA 37 E (BASF AG) | PVP/VA Copolymer | - | - | - | - | - | 3,0 | - | - |
| Merquat 550 (Ondeo) | Polyquaterinium-7 | 0,5 | 1,0 | - | - | - | - | - | - |
| Mineral Oil | Mineral Oil | - | - | - | - | - | - | 88,8 | 3,0 |
| Mulsifan RT 203/80 (Z&S) | C12-15 Pareth-12 | - | - | - | - | 4,0 | - | - | - |
| Neo Heliopan AV (Symrise) | Ethylhexyl Methoxycinnamate | - | - | - | - | - | - | 0,5 | - |
| Neo Heliopan BB (Symrise) | Benzophenone-3 | 0,1 | 0,1 | - | - | - | 0,2 | - | - |
| Neo Heliopan Hydro (Symrise) | Phenylbenzimidazole Sulfonic Acid | - | - | - | - | - | 0,2 | - | - |
| Neo-PCL Water soluble N (Symrise) | Trideceth-9, PEG-5 Ethylhexanoate, Water (Aqua) | 1,0 | - | - | - | - | - | - | - |
| Neutrol TE (BASF) | Tetrahydroxypropyl Ethylendiamine | - | - | - | - | 1,0 | - | - | - |
| Octopirox | Piroctone Olamine | 0,5 | 0,5 | - | - | - | - | - | - |
| Polyquart H-81 (Cognis) | PEG-15 Cocopolyamine | - | - | 3,0 | - | - | - | - | - |
| PCL Liquid 100 (Symrise) | Cetearyl Octanoate | - | - | - | 0,5 | - | - | - | - |
| Propylenglycol | Propylene Glycol | - | - | - | - | - | - | - | 5,0 |
| Rose CL forte (Symrise) | Water (Aqua), Glycerin, PEG-40 Hydrogenated Castor Oil, Rosa Damascena Flower Oil | - | 0,5 | - | - | - | - | - | - |
| Schwefel | Sulfur | 0,1 | - | - | - | - | - | - | - |
| Selendisulfide | Selenium Sulfide | - | - | 1,0 | - | - | - | - | - |
| Sensiva SC 50 | Ethylhexylglycerin | - | - | - | - | 0,5 | - | - | - |
| Natriumchlorid | Sodium Chloride | 0,8 | 0,5 | - | - | - | - | - | - |
| Natronlauge, 20%ig | Sodium Hydroxide | 0,2 | 0,1 | - | 0,2 | - | - | - | 8,0 |
| Solubilizer (Symrise) | PEG-40 Hydrogenated Castor Oil, Trideceth-9, Propylene Glycol, Water (Aqua) | - | - | - | - | - | 2,0 | - | - |
| Symdiol 68 (Symrise) | 1,2 Hexanediol, Caprylylglycol - | - | - | - | - | 1,0 | - | - | - |
| Symrise Fragrance | Fragrance | 0.3 | 0.3 | 0.3 | 0.2 | 0.4 | 0.2 | 0.5 | - |
| Tetradecandiol, 1,2 | Tetradecandiol, 1,2 | - | 0,1 | 0,5 | - | - | 0,2 | 0,5 | 1,0 |
| Texapon N 70 (Cognis) | Sodium Laureth Sulfate | - | 10,0 | - | - | - | - | - | - |
| Undecylensäure | Undecylenic Acid | 0,2 | - | 0,3 | - | - | - | - | - |
| Vetikol (Symrise) | Dimethyl Phenyl 2-Butanol | - | - | - | - | - | 1,0 | - | - |
| Water, demineralisiert | Wasser (Aqua) | Ad 100 | Ad 100 | Ad 100 | Ad 100 | Ad 100 | Ad 100 | Ad 100 | Ad 100 |
| Zinkpyrithion | Zinc Pyrithione | 1,0 | - | 0,5 | - | - | - | - | - |

| | | | | | | | | | |
|---|---|---|---|---|---|---|---|---|---|
| 1 = Anti-Schuppen Shampoo 2 = 2 in 1 Anti-Schuppen Shampoo 3 = Hair Conditioner gegen Schuppen, leave on 4 = Hair Conditioner gegen Schuppen, rinse off 5 =Anti-Schuppen Haarfestiger Gel 6 = Anti-Schuppen Haarpflegespray 7 = Haaröl gegen Schuppen 8 = Anti-Schuppen Entkräuselungscreme | | | | | | | | | |

## Patentansprüche

1. Verwendung von ein, zwei, drei oder mehreren C-10-C14-Alkan-1,2-diolen zur Herstellung eines Mittels zur Prophylaxe und/oder Behandlung von Malassezia-induzierter Schuppenbildung.

2. Verwendung gemäß Anspruch 1, **dadurch gekennzeichnet, dass** eine Verbindung oder eine Mischung aus zwei oder drei Verbindungen aus der Gruppe bestehend aus 1,2-Decandiol, 1,2-Dodecandiol und 1,2-Tetradecandiol ausgewählt wird.

3. Topisch zu applizierende, kosmetische und/oder dermatologische Zubereitung umfassend oder bestehend aus:
a) einer Schuppen-reduzierenden Menge an ein, zwei, drei, vier oder mehreren C10-C14-Alkan-1,2-Diolen,
b) einer Schuppen-reduzierenden Menge an ein, zwei, drei, vier oder mehreren Antischuppen-Mitteln ausgewählt aus der Gruppe bestehend aus Climbazol, Benzimidazol, Benzothiazol, Bifonazol, Butaconazolnitrat, Clotrimazol, Croconazol, Eberconazol, Econazol, Elubiol, Fenticonazol, Fluconazol, Flutimazol, Isoconazol, Ketoconazol, Lanoconazol, Metronidazol, Miconazol, Neticonazol, Omoconazol, Oxiconazolnitrat, Sertaconazol, Sulconazolnitrat, Thioconazol, Itraconazol, Zinkpyrithion, Steinkohleteer, Schwefel, Selendisulfid, Aluminumchlorid, Octopirox, Cyclopiroxolamine, Undecylensäure und deren Metallsalze, Harnstoffderivate, Griseofulvin, 8-Hydroxyquinolin, Ciloquinol, Thiobendazol, Thiocarbamate, Triclosan, Haloprogin, Polyene, Hydroxypyridon, Morpholin, Benzylamin, Allylamine, Teebaumöl, Nelkenblätteröl, Korianderöl, Palmarosaöl, Thymianöl, Zimtöl sowie etherisches Öl der Bitterorange, Zimtaldehyd, Citronellsäure, Farnesol, Berberin, Hinokitiol, Tropolon, Birkenteeröle, Ichthyol, Sensiva SC-50, Polyglycerolester, Arylalkylalkohole, 3-Phenyl-1-propanol, Vetikol, Muguetalkohol, Elestab HP-100, Azelainsäure, Lyticas, Isothiazolinone und lodopropinylbutyl-carbamat und
c) gegebenenfalls ein, zwei oder mehreren Hilfs- und/oder Zusatzstoffen.

4. Zubereitung gemäß Anspruch 3, **dadurch gekennzeichnet, dass** ein, zwei oder drei C10-C14-Alkan-diole ausgewählt werden aus der Gruppe bestehend aus 1,2-Decandiol, 1,2-Dodecandiol und 1,2-Tetradecandiol.

5. Zubereitung gemäß Anspruch 3 oder 4, **dadurch gekennzeichnet, dass** ein, zwei oder mehrere Hilfs- und/oder Zusatzstoffe aus der Gruppe der Riechstoffe ausgewählt werden.

6. Duftstoffkomposition umfassend oder bestehend aus:
a) einer sensorisch wirksamen Menge eines Riechstoffes oder der Summe aus zwei, drei, vier oder mehreren Riechstoffen,
b) einer Schuppen-reduzierenden Menge eines, zweier, dreier oder mehrerer, bevorzugt eines, zweier oder dreier C10-C14-Alkan-1,2-diole und
c) gegebenenfalls ein oder mehreren Träger- und/oder Zusatzstoffen.
